# EUROPEAN PATENT APPLICATION

(11) **EP 3 326 527 A1**
(43) Date of publication of application: **30.05.2018**
(21) Application number: 16200135.8
(22) Date of filing: 23.11.2016
(51) Int. Cl.: A61B 5/11, A61B 5/00, G06T 7/00

(54) **METHOD AND ARRANGEMENT FOR ELECTROMAGNETIC RADIATION BASED NON-INVASIVE MONITORING OF A PERFORMANCE OF AN ANATOMIC OBJECT DURING AN OPERATION OR MEDICAL INTERVENTION**

(71) Applicant: TTY-säätiö sr, 33101 Tampere (FI)
(72) Inventor: HOKKA, Mikko, 14500 Iittala (FI); CURTZE, Sven, 00220 Helsinki (FI); KUOKKALA, Veli-Tapani, 33310 Tampere (FI)
(74) Representative: Berggren Oy, Helsinki & Oulu

(57) **Abstract**

An arrangement (100) for electromagnetic radiation based non-invasive monitoring or assessment of a performance of an anatomic object, such as a heart (101) during an operation, such as an open cardiac surgery of a subject comprises at least one imaging device (102) or imaging input device for obtaining at least two or more images of at least one surface point (113) on a surface of the anatomic object over at least one fraction of a characteristic movement cycle from the surface of the anatomic object. In addition the arrangement comprises an output for a display device (106) or said display device (106), and a control unit (104) for determining deformation based on movements of said at least one surface point (113) over said at least one fraction of the cycle between said at least two or more images of the surface of the heart in function of time. Further changes in said deformation is determined or determined deformation is compared to reference deformation values to find any deviation in said performance or state of the deformation determined. Additionally control data is provided for controlling the display device (106) to display the changes in said deformation or said deviation or state of the deformation determined in function of time.

## Description

### TECHNICAL FIELD OF THE INVENTION

The invention relates to a method and arrangement for electromagnetic radiation based non-invasive monitoring or assessment of a performance of an anatomic object, such as a heart, during an operation or medical examination. In particular, the invention relates to the method and arrangement for determining deformation of the heart during an open cardiac surgery, and especially a right side or a right ventricle (RV) of the heart during the open cardiac surgery in order to assess or estimate the performance of the heart.

### BACKGROUND OF THE INVENTION

Determining the performance of an anatomic object during an operation (but also in non-operative risk stratification) is often an important topic to be assessed. For example, the determining of the performance of the heart (or myocardium, which is a heart muscle) during the open cardiac surgery and therapy, such as a volume therapy, is very important in order to discover and notice possible dysfunctions of the heart, like heart failure or heart attack. Currently, there is no gold standard existing for example for right ventricle (RV) performance assessment during open cardiac surgery. Many functions and dysfunctions of the right ventricle of the heart are assessed using an "eyeballing" routine, i.e., the clinician judges visually based on his/her experience if the movement of the ventricle looks right or wrong, which is of course not very accurate and depends purely on the clinician and his/her experience or even his/her alertness and in addition the assessment is not often even repeatable.

In addition, direct and/or indirect measurements of the right ventricle preload (Volume therapy) are known in order to make an assessment. The indirect measurements of CVP (central venous pressure) using a Central Venous Catheter are typically used to predict the success of volume therapy. However, this does not necessarily relate to the condition or functioning of the heart. Central Venous Pressure (CVP) using a Central Venous Catheter has been, and often still is, used as a surrogate for preload or end-diastolic volume (EDV), and changes in CVP in response to infusions of intravenous fluid have been used to predict volume-responsiveness (i.e. whether more fluid will improve cardiac output). However, there is increasing evidence that CVP, whether as an absolute value or in terms of changes in response to fluid, does not correlate with ventricular volume (i.e. preload) or volume-responsiveness, and so should not be used to guide intravenous fluid therapy. All patients undergoing heart surgery belong to various high risk categories, and therefore, the safe levels of preload can vary from patient to patient significantly. Another method currently used to obtain information about preload is transesophageal echocardiography (TEE), which works well in imaging the thoracic aorta but does not provide good signals from the right ventricle.

The previously used methods include also at least various forms of Doppler ultrasound methods, various forms of Echocardiography, and intravenous catheterization. Also the ejection fraction is commonly measured by echocardiography in order to make any assessment in which the volumes of the heart's chambers are measured during the cardiac cycle. The ejection fraction can then be obtained by dividing the volume ejected by the heart (stroke volume) by the volume of the filled heart (end-diastolic volume). The ejection fraction can also be measured by computed tomography (CT scan), magnetic resonance imaging (MRI), ventriculography, gated SPECT and radionuclide angiography (MUGA) scanning. A MUGA scan involves the injection of a radioisotope into the blood and detecting its flow through the left ventricle. Historically, the gold standard for measurement of the ejection fraction is ventriculography. However, none of these methods are especially feasible for the right heart assessment during the operation, because they are either intrusive (touch the patient), and cannot thus be used during the surgery (perioperative or during the operation), use hazardous radiation (e.g. X-Rays), cannot be focused easily to the right ventricle, are too bulky to fit into the operating theatre and at the same time are often too cost inefficient when bound to the operating theatre.

Currently, only echocardiography can provide some information about the longitudinal right ventricular deformation. This method is, however, in most cases not very well suited for right heart imaging and analysis during the operation since the probe must be placed in the esophagus of the patient, which poses strong geometrical constraints in combination with high level of user skills. The best available method at the moment is still a visual evaluation ("eyeballing") by the operating surgeon or anesthesiologist, but it still has its shortcomings as described above.

In addition also determining the performance of other anatomic objects is important, such as determining lungs e.g. during a medical operation or muscles, like back muscles or limb muscles e.g. during orthopaedic operations or medical examination, which are also determined and assessed visually and thus have the same problems as discussed above.

### SUMMARY OF THE INVENTION

An object of the invention is to alleviate and eliminate the problems relating to the known prior art. Especially the object of the invention is to provide an easy, fast, accurate and safe method for assessing the performance of the anatomic object, and especially the heart during the operation, and especially the right side or a right ventricle (RV) of the heart during the open cardiac surgery so that the monitoring and assessment can be done in a reliable and non-invasive way and not using any dangerous or harmful ionizing radiation.

The object of the invention can be achieved by the features of independent claims.

The invention relates to an arrangement for electromagnetic radiation based non-invasive monitoring and assessment of a performance of an anatomic object during an operation or the medical examination of a subject according to claim 1. In addition, the invention relates to an imaging device according to claim 18, a corresponding method of claim 19 and computer program product of claim 20.

According to an embodiment of the invention a monitoring and assessment of a performance of an anatomic object during an operation or medical examination of a subject (so a patient) is performed non-invasively and using electromagnetic radiation. Advantageously optical or near optical electromagnetic radiation cameras can be used, such as cameras using a CCD, CMOS or other known and suitable technology.

More particularly, the monitoring and assessment of performance relates to determining deformation, like absolute and/or relative deformation, of a muscle during an operation, such as during surgery, orthopaedic operation or examination, or during other operation, where a tracking of any muscle deformation under defined movement sequences is important.

Next examples are related especially to embodiments for determining and tracking deformations of a myocardium so the heart deformations. However, it is to be noted that still these embodiment are applicable for determining and tracking also other kinds of anatomic or muscle deformations, such as the lungs' deformation due to breathing or muscle deformations due to muscle movements.

In particular an embodiment of the invention for monitoring and assessment of performance relates to determining deformation, like absolute and/or relative deformation, of a right side or a right ventricle (RV) of the heart during the open cardiac surgery of the subject, and additionally, determining changes in said deformation in a function of time, so over a fraction or period of a cardiac cycle or several cardiac cycles. Should there occur any deviation, which is outside an acceptable and predetermined range or limits of deviation, or should the state of the deformation determined be outside an acceptable and predetermined range or limits of deviation, an indication is advantageously provided. Alternatively, or in addition to, also a control signal can be provided. The control signal can be a signal for controlling an indication device, such as a display device or the like display an indication, or the control signal can be a signal for controlling a life supporting machine used for the patient in question in order to change the operation parameters of the life supporting machine.

The indication can be a signal displayed on a display or alarm type indication or other indication known by the skilled person. Also a trend line representing a trend of changes in said deformation can be provided. In addition the value of the deformation and/or a trend of the changes in said deformation can be compared to a predetermined value(s) or range, and whether said determined deformation or a trend deviates from the predetermined value or range, an alarm or other indication indicating the deviating values and/or changes can be provided. These examples offer clear and legible indications, e.g., to operators, such as clinicians during the operation, whereupon they can be more focused on the operation as such.

In particular, it is to be noted that the indication can be visualized information or data on a two or three dimensional display device, such as a screen, projector, mixed or augmented reality smart glasses or other optical head-mounted displays, for example. In addition the indication can be in a form of indicative values, charts, and 2D or 3D-color coded data maps optionally overlain on top of original image data. All measures can be digitally stored, exported and attached to the surgery minutes, providing quantified records of perioperative patient condition, which is clear advantage in view of the prior art "eyeball" methods, for example.

According to the embodiment at least two or more images, advantageously sequential images, of at least one surface point on a surface of the heart is obtained over at least one fraction or period of a cardiac cycle or several cardiac cycles. The images can be obtained in defined time intervals Δt. In addition, at least one surface point can be imaged, but according to an advantageous example a fixed pattern is provided on the surface of the heart, such as to the right side or right ventricle (RV) of the heart. The fixed pattern on the surface of the heart can be provided, e.g., by marking with ink, a stencil, a stamp, transfer paper, or advantageously a film having the pattern or suitable markings, such as a suitable unstructured grid or pattern, can be used. The film is advantageously a film which can be attached on the surface of the heart so that it can be removed easily after the usage. Also a natural tissue texture, anatomic feature, or other point, such as a point of a blood vessel on the surface of the heart can be tracked.

Deformations of the surface of the heart are then determined advantageously by tracking movements of said at least one surface point over said at least one fraction or period of the cardiac cycle between at least two or more images of the surface of the heart in function of time. In order to find any deviation in said performance changes said deformation is determined or determined deformation is compared to reference deformation values to estimate whether the changes in said deformation or state of the deformation determined is in an acceptable level or range. Based on the output the suitable indication or control data can be provided. According to an embodiment control data for controlling the display device can be provided to display the changes in said deformation or said deviation or state of the deformation determined in function of time.

In addition, according to an advantageous embodiment of the invention control data can be provided based on said determination(s) also for controlling e.g. life support machines, equipment and/or measures so that the determined changes in said deformation is kept or adjusted in a predetermined range or value, or a target range or value is again achieved. The life support machines, equipment and/or measures to be controlled are for example cardiopulmonary bypass pump settings, ventilator or respirator settings (volume, pressure, flow) or infusion pumps. However, it is to be noted that these are only examples and also other equipment, arrangements and devices can be controlled. The control data advantageously controls the operation parameters of the life supporting machines so to change the operation of the machine in question. For example if a volume therapy is applied and fluids are given at a higher rate or in a larger volume than the system can absorb or excrete, it can be detected by determining the deformations and thereby any changes in said deformations. Thus, if there exists any changes in said deformations during the volume therapy so that said changes are over the limits or whether the state of the deformation is not in the acceptable range, the fluid rate in the volume therapy can be manipulated by the control data provided so that the desirable deformation is achieved, so in this example either increasing or decreasing the fluid rate infused.

According to an embodiment the deformation is or comprises at least one of the following:
- variable distance of at least two surface points or local displacement of at least one surface point on the surface of the right side of the heart over said at least one fraction of the cardiac cycle,
- tissue displacement, velocity, or acceleration of at least one surface point over said at least one fraction of the cardiac cycle, which defines the movement of a surface point of the right side of the heart,
- contractility indicator based on strain rate, shortening fraction, principal shortening strain or other deformation values determined from at least two surface points over said at least one fraction of the cardiac cycle, or
- contractility based on said contractility indicator and simultaneous measurement of pressures in a right atrium or right ventricle with a pulmonary artery catheter or other pressure measurement, and/or
- variable surface area and/or volume defined by at least three surface points on the surface of the heart over said at least one fraction of the cardiac cycle.

According to an advantageous embodiment, the displacement vector(s) is provided for at least one surface point on the images as a time series function from the data representing an arrangement of pixel kernels in space for determining said changes in said deformation. It is to be noted that due to the change of location and shape of the object, the position of pixel kernels in space changes over time, from which the displacement vectors of surface points can be determined as a time series function at sub-pixel resolution.

According to an embodiment, external time series data can be gathered for use as a trigger pulse or a common time base for synchronizing or linking at least two or more (sequential) images and/or or other measurement data from at least one of said imaging device or other device with each other. Using the trigger pulse or a common time base allows for determining the changes in said deformation between said two or more (sequential) images either in combination or together with said other (external) measurement data. Said external time series data can be gathered e.g. from at least one additional external measuring device measuring or monitoring vital signs of the subject so the patient undergoing open cardiac surgery. An example of the external measuring device is e.g. an electrocardiography (ECG) device, but naturally it can also be any other suitable device measuring vital signs, from which the time series data can be gathered, such as devices for measuring or determining heart rate, blood pressure, central venous pressure, pulmonary artery pressure, pulmonary artery occlusion pressure, inspired and expired gases, oxygen saturation of the blood, cardiac output, ultrasound or tissue doppler imaging data or other devices for accurate monitoring and quantification of cardiac cycle events.

The trigger pulse or the common time base based on the (external) time series data can also be used as a gating signal. For example a zero-displacement reference frame can be set for a first image gated by the trigger pulse or the common time base. After this a displacement vector provided for at least one second subsequent image obtained over at least one next fraction of a cardiac cycle can be compared to the zero-displacement reference frame, which allows determining said deformation or comparison with the reference deformation values or changes in said deformation. In addition, the trigger signal from e.g. an ECG monitor or other suitable measuring vital signs can be received, whereupon said trigger signal can be used for starting data acquisition in case of detecting abnormal electrical activity, for example.

Extracting displacement data time series functions x→f(t) enables visualization and quantification of the deformation pattern characteristics with respect e.g. to amplitude, amplitude modulation, periodicity, aperiodicity, wavelength, frequency, phase, rhythm and/or combination thereof for representing said deformation or said changes in said deformation, which might be associated with certain diseases or conditions.

In addition, a time lag between an event of an electrical activity measured e.g. by electrocardiography (ECG) or other device and the deformation pattern for representing said deformation or said changes in said deformation can be determined, describing or assessing mechanical activity of the heart. The time lag might be possibly associated with certain diseases or conditions.

According to an embodiment the state of the deformation and/or changes in said deformation can be determined by identifying common points or pixel kernels on images captured by at least two different imaging devices at the same point in time, and by tracing on each image of an imaging time sequence (3D space), where said tracing or a correlation is performed on images. It is to be noted that the unique intensity distribution features within each pixel matrix of defined matrix size originate either from natural tissue texture or can be artificially applied, as previously discussed in connection with the providing the patterns or markings on the surface of the heart.

Furthermore, data related to the deformation or changes in said deformation can be communicated to a database, such as a cloud system or other external service, as an example. By this, for example, the determined deformation or changes in said deformation data can be compared to data previously stored in the database. Thus possible matches with the previously stored data can be identified and a possible association with certain diseases or conditions inducing deformation and/or deviating changes in said deformation can be found, such as e.g. valvular heart disease or problem with a mitral valve, tricuspid valve, ventricle or atrium, volume or pressure overload, arrhythmia, dysfunction, toxicity, ischemia, energy depletion, for example. Advantageously, if any match is found, its reliability can be estimated for example by comparing how good the match is expressed as percentage value, and the match, as well as its reliability, can be displayed to the operators.

Even if the examples above are related to heart performance determination, they are suitable for determining and tracking also other kinds of anatomic or muscle deformations, such as lungs deformation due to breathing or muscle deformations due to muscle movements. The deformation and thereby the performance of the lungs can be determined in a similar manner as the performance of the heart is explained above. The lungs can be determined for example during a medical operation, whereupon any abnormal operation can be noticed in a similar manner as by the embodiments discussed elsewhere in this document. In addition the deformation and thereby the performance of the muscles can be determined in a similar manner than the performance of the heart is explained above. The muscles can be determined for example during an orthopaedic operation, whereupon any abnormal operation can be noticed in a similar manner as by the embodiments discussed elsewhere in this document. However, it is to be noticed that for example the deformations of the lungs are cyclic due to autonomous breathing (even if the breathing can be controlled also consciously), whereas the deformations of the muscles must be carried out consciously, such as for example by bending an upper body alternatively to the left and right or backwards and forwards, whereupon for example differences between the deformations and thereby performances of the left and right muscles can be determined by the embodiments of the invention by imaging the points applied on the surface of the muscles or on the skin and thereby tracking the muscle movement under defined movement sequences.

The present invention offers advantages over the known prior art, such as enabling visual light spectrum based or near visual light electromagnetic radiation based non-invasive measurements of the absolute and relative deformation of the right side of the heart, clinical presentation, assessment, and diagnosis of the function and condition of the heart as well as treatment guidance during open heart surgery with less effort and with greater accuracy than state-of-the-art procedures.

In particular, the present invention offers a non-invasive and non-contact method for monitoring and assessing the performance or condition of the heart, and additionally without the use of hazardous radiation. For example, the currently used transesophageal echocardiogram (TEE), on the other hand, is the only imaging technique used during open cardiac surgery, but this, however, requires a probe to be passed into the patient's esophagus with the risk of esophageal perforation. In addition, the imaging of the right ventricle with the TEE is very difficult due to signal dispersion or signal weakening caused during propagation through and by interaction with body tissue. However, the signals utilized by the current invention are not subject to these constraints.

Furthermore the technique of the present invention is less time consuming when compared to TEE and the spatial resolution is much more improved. The current invention offers also a user and experience independent method as opposed to highly user and experience dependent TEE analysis. Moreover the current invention offers quantifiable measures vs. routinely used cognition based visual observation. The digital proof and documentation of patient condition during the operation is also possible as opposed to vague wording.

The embodiments of the invention deliver measures for tissue velocity, strain rate, shortening fraction/principal shortening strain etc., which are direct indicators for ventricle contractility. When combined with load measurements such as delivered by a Pulmonary Artery Catheter or other measurement means, direct contractility measures can be obtained via the relationship of load and strain. In addition, simultaneous acquisition, e.g., with ultrasound imaging modalities allows for improvements in right ventricle wall movement assessment and 3D-model generation. Simultaneous acquisition and deformation pattern analysis using tissue Doppler ultrasound, spackle tracking, or any other ultrasound based deformation analysis focusing on the left side of the heart allows for accurate identification and quantification of ventricular cycle timing and detection of dys-synchrony or asynchrony between right and left side of the heart.

In addition, the present invention quantifies deformation, local displacement, tissue velocity, strain, strain-rate, and acceleration, as function of time, enabling time series data pattern recognition and amplitude variation detection, and thus supporting performance assessment of the condition and function of the heart based on the following features:
- uses optical photography and white or other colour light, or other imaging based on electromagnetic waves,
- allows measurements of local deformation instead of only global deformation at high spatial and time resolution (theoretically pm vs. mm in ultrasound), and delivers accurate quantitative and relatively comparable measures,
- can be used to assist anesthesia related procedures when assessing the effect on the right side of the heart, such as ventilation pressure, fluid therapy, or medication,
- fast and repeatable,
- allows storage of quantitative parameters and their attachment to the surgery minutes, whereas currently clinicians struggle to put their observations into words,
- pattern recognition can be automated and detect irregularities in the heart's movement,
- can deliver a variety of measures which clinicians are familiar with from techniques such as ultrasound, partly at higher quality; learning and understanding of the technique is easy with understanding of established techniques, and
- can be limited to the visible fraction of the heart in the opened pericardium, however, usually the visible fraction is sufficient to obtain the relevant data and also the currently used "eyeballing" routine is limited to the same fraction.

Cardiac Output is difficult to measure and there is no gold standard to compare the different measurement techniques against. The new method provides an indirect non-contact method for obtaining cardiac output for the right ventricle, which can be compared with other methods. There are a number of clinical methods to measure cardiac output, ranging from direct intracardiac catheterization to non-invasive measurement of the arterial pulse. Each method has advantages and drawbacks. Relative comparison is limited by the absence of a widely accepted "gold standard" measurement. Cardiac output can also be affected significantly by the phase of respiration - intra-thoracic pressure changes influence diastolic filling and therefore cardiac output. This is especially important during mechanical ventilation, in which cardiac output can vary by up to 50% across a single respiratory cycle. Cardiac output should therefore be measured at evenly spaced points over a single cycle or averaged over several cycles. Invasive methods are well accepted, but there is increasing evidence that these methods are neither accurate nor effective in guiding therapy. Consequently, the focus on development of non-invasive methods is growing. The present invention can derive values correlating with cardiac output and perform a deconvolution of the contribution of respiration activity to the overall measured cardiac output, resulting in more precise measures.

For example ejection fraction (EF) is an important determinant of the severity of systolic heart failure. Damage to the muscle of the heart (myocardium), such as that sustained during myocardial infarction or in atrial fibrillation or a plurality of etiologies of cardiomyopathy, compromises the heart's ability to perform as an efficient pump (ejecting blood) and, therefore, reduces ejection fraction. This reduction in the ejection fraction can manifest itself clinically as heart failure. Unlike heart rate, which can be high or low in a healthy person and can vary over the course of the day, a low ejection fraction is always associated with disease. Current technology can provide measurements of the ejection fraction of the left ventricle, but only intravenous catheterization (intrusive) can provide information about ejection fraction of the right ventricle. The present solution enables measurements of fractional shortening (principal shortening strain) values and conversion to EF values if the lengths of the ventricle has been determined priory via ultrasound measurements, providing therefore a non-invasive determination of RVEF.

Right ventricular dysfunction (the ventricles ability to pump blood) is associated with a significant increase in mortality in cardiac surgery. Early recognition of right ventricular failure is important. Currently, the dysfunction can be detected only by correct interpretation of TEE and hemodynamic data. The current invention allows quantification of the function of the right heart and fast identification of the right ventricular dysfunction. Diastolic dysfunction is manifested in a higher stiffness of right ventricle tissue with higher resistance to filling, resulting in distinct wall and tissue movement patterns observable with the present invention.

Systolic dysfunction is manifested in reduced contractility of the ventricle, which can be visualized by reduced regional wall motion using the embodiment of the present invention. Measurement of Ventricle and Atrium contraction timing and synchrony, assisting the setting of AV (Atrium and Ventricle) pacemaker interval timing as well as in maximizing atrial function during the weaning stage is also possible. Moreover, naturally, the screw-like deformation of the heart propagates from top-to-bottom (base to apex), but with a pacemaker attached to the apex, it is vice versa. According to the current invention, local deformation can be analyzed and compared with and without pacemaker, and assist in deciding whether a pacemaker is beneficial or even detrimental.

Currently there are no feasible methods for quantitative measurements that allow for perioperative investigation. The current invention can measure the deformation of the right ventricle in all three principal directions; normal, circumferential, and longitudinal directions. In particular, the right ventricular longitudinal strain (RVLS) is an important measure that correlates, for instance, with myocardial fibrosis in patients with end-stage heart failure.

In addition, for example during valve replacement surgeries of both the tricuspid valve and mitral valve, surgeons take cross-sectional, circumferential and other measures at specific positions of the ventricles in order to choose the correct replacement valve size. The tools used are physical measurement tools. However, holding the tools in their hands, often whilst simultaneously handling other tools or manipulating the position of the heart can be difficult. The current invention provides also a solution to measure distances and angles contact free on a digital image.

The invention overall delivers high spatial and time resolution measurements of tissue displacement, enabling precise and detailed detection of cardiac cycle events, including isovolumic relaxation and contraction of the right ventricle. The data obtained is comparable and complementary to data obtained by Ultrasound Tissue Doppler or Spackle Tracking for the left ventricle (LV).

The exemplary embodiments presented in this text are not to be interpreted to pose limitations to the applicability of the appended claims. The verb "to comprise" is used in this text as an open limitation that does not exclude the existence of also unrecited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figure 1: illustrates a principle of an exemplary arrangement for monitoring and assessment of a performance of a heart during an open cardiac surgery of a patient according to an advantageous embodiment of the invention,
- Figure 2: illustrates an exemplary method providing displacement vectors of the images in order to assess deformations and changes in the deformations according to an advantageous embodiment of the invention,
- Figure 3: illustrates an exemplary method for providing trigger pulse or a common time base according to an advantageous embodiment of the invention,
- Figure 4: illustrates an exemplary amplitude of monitored deformation change of the surface of the heart during a volume therapy according to an advantageous embodiment of the invention, and
- Figures 5-7: illustrate examples of displacement and deformation data time series according to an advantageous embodiment of the invention.

### DETAILED DESCRIPTION

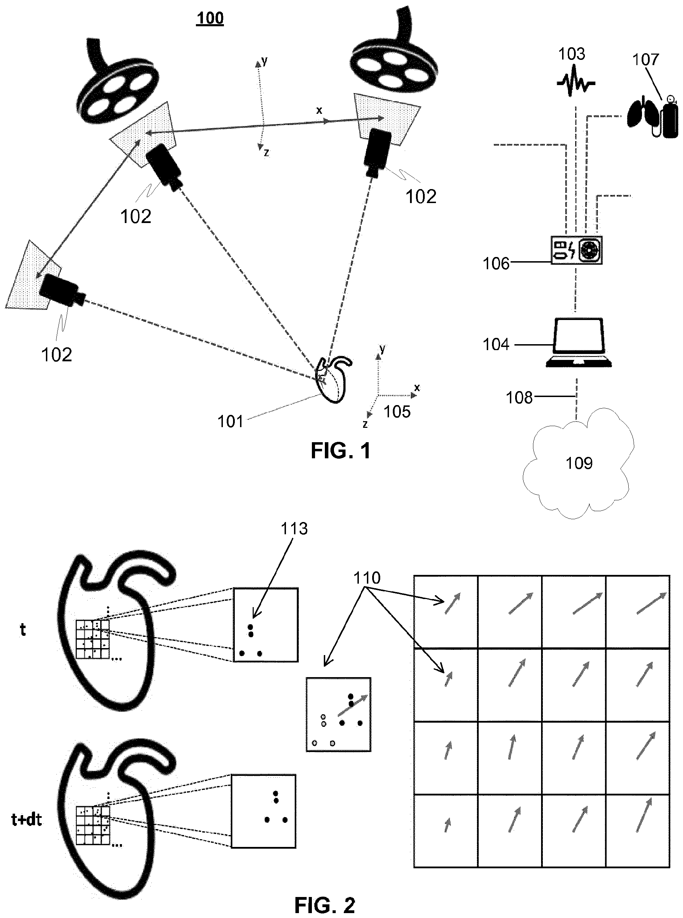

Figure 1 illustrates a principle of an exemplary arrangement 100 for monitoring and assessment of a performance of a heart 101 during an open cardiac surgery of a patient according to an advantageous embodiment of the invention. The arrangement 100 comprises imaging device 102, such as digital optical or other electromagnetic wave cameras (1, 2, or more), which can be CCD, CMOS, or other technology. The camera(s) is/ are placed in three dimensional space at a/different position(s) (each) with a defined line of sight or radiation propagation direction to/from the object (heart). The camera position(s) in relation to the object and to each other are advantageously defined via a calibration procedure using a physical calibration object with defined geometrical features positioned at the intersection point of the different lines of sight/ rays or by in-situ bundle adjustments, or other methods. Polarizing filters (not shown) can be placed in front of the OR-lights or dedicated polarized light sources can be used as well as polarizing filters in front of the camera to extinguish reflections or glare from the surface of the object.

The imaging devices 102 are configured to take two or more photographic images over the period of a fraction of a cardiac cycle or several cardiac cycles in defined time intervals Δt, whilst advantageously simultaneously synchronized time series data from an electrocardiography device and/ or other external devices 103 measuring vital signs of the subject (patient undergoing open cardiac surgery) can be acquired. Camera timing is controlled - and camera data captured via an electronic controller unit or processing unit 104 equipped with real time image capturing hardware whilst other external signals are captured digitally, using the dedicated communication protocols of the external devices, or analogue, using analogue-to-digital converters, where one of the signals can act as gating signal. Data is either processed after transfer to a computer unit, e.g., via Ethernet, or using an embedded graphics processing unit (GPU), as an example. In addition the arrangement 100 comprises advantageously a display device 106.

The processing unit 104 is advantageously configured to determine (by tracking) the deformation based on movements of surface points over at least one fraction of the cardiac cycle between said at least two or more images of the surface of the heart in function of time. In addition the processing unit 104 is configured to determine changes in the deformation to find any deviation in the performance or state of the deformation determined (or a feature changing during a cycle but in a normal state being regular and sequentially repetitive over several cycles). Alternatively determined deformation can be compared to reference deformation values to make said findings. Advantageously the processing unit 104 provides control data for controlling the display device 106 to display the changes in said deformation or said deviation or state of the deformation determined in function of time. The processing unit 104 can also provide control data or signal for controlling a life supporting machine 107 as is described elsewhere in this document.

In addition, the arrangement 100 may comprise or is at least arranged in a data communication 108 with a database 109, such as a cloud or other external service. Data related to, e.g., the deformation can be provided to the database, wherein said data can be compared to previously stored data in said database and thereby possible match with the previously stored data can be identified and possible associations with certain diseases or conditions inducing said deformation and/or deviating changes in said deformation found, such as e.g. valvular heart disease or problem with a mitral valve, tricuspid valve, ventricle or atrium, volume or pressure overload, arrhythmia, dysfunction, toxicity, ischemia, energy depletion, for example.

Figure 2 illustrates an exemplary principle for providing displacement vectors 110 of the images in order to assess deformations and changes in the deformations according to an advantageous embodiment of the invention. The two- or three-dimensional coordinates 105 of points on the object surface are determined by measurements made with the camera(s) using a coordinate system transformation. Common points or pixel kernels are identified on images captured by the different cameras at the same point in time, and traced on each image of an imaging time sequence, where tracing or correlation can be performed on consecutive images or nonconsecutive images, as can be seen in Figure 2. The unique intensity distribution features within each pixel matrix of defined matrix size originate either from natural tissue texture or can be artificially applied. Due to the change of location (caused by respiration/ ventilation of the subject and rigid body movement of the object due to competition between right and left ventricle for space in the pericardium as well as cardiopulmonary interaction) and shape (caused by cardiac activity) of the object, the position of pixel kernels in space changes over time *t*, from which the displacement vectors 110 of surface points can be determined as a time series function at sub-pixel resolution (translating into spatial sampling frequency of several micrometers) at time resolution between microseconds up to seconds. The above described Digital Image Correlation routine can herein refer to feature tracking, intensity tracking, or any other means of image registration or image alignment algorithms, where the trade-off is between processing time and spatio-temporal resolution.

According to an advantageous embodiment displacement vector(s) 110 is provided for at least one surface point 113 on the images as a time series function at sub-pixel resolution from the data representing a position of pixel kernels in space for determining said changes in said deformation. It is to be noted that due to the change of location and shape of the object, the position of pixel kernels in space changes over time, from which the displacement vectors of surface points can be determined as a time series function at sub-pixel resolution.

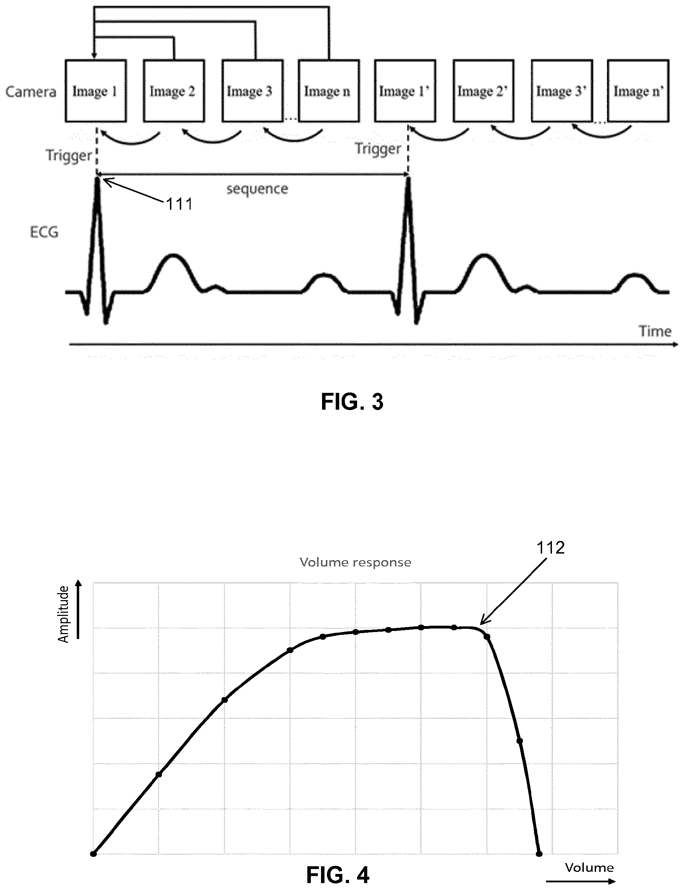

Figure 3 illustrates an exemplary method for providing and using a trigger pulse 111 or a common time base according to an advantageous embodiment of the invention. The external time series data, such as ECG, can be gathered and used as said trigger pulse 111 or a common time base for synchronizing or linking at least two or more (sequential) images and/or or other measurement data from at least one of said imaging device or other device with each other. Using of the trigger pulse 111 or a common time base allows determining the changes in said deformation between the two or more (sequential) images either in combination or together with said other (external) measurement data. Said external time series data can be gathered, e.g., from at least one additional external measuring device measuring or monitoring vital signs of the subject so the patient undergoing open cardiac surgery. An example of the external measuring device is, e.g., an electrocardiography (ECG) device, but naturally it can also be any other suitable device measuring vital signs, from which the time series data can be gathered, such as devices for measuring or determining heart rate, blood pressure, central venous pressure, pulmonary artery pressure, pulmonary artery occlusion pressure, inspired and expired gases, oxygen saturation of the blood, cardiac output, ultrasound or tissue doppler imaging data or other devices for accurate monitoring and quantification of cardiac cycle events.

For an object changing its shape (and position) over time (such as heart beating), where the deformation cyclically increases and decreases as function of time t, the correlation coefficient r of the cross correlation function normally decreases with increasing deformation, making the determination of displacement vectors more inaccurate and prone to bias when using a fixed reference image. For a series of n images, the cross correlation coefficient r is typically minimized for consecutive image pairs n/n+1, which is utilized in the sum of differentials routine. However, errors in the determination of displacement vectors cumulate when using this routine, and the cumulative error therefore increases with increasing length of the measurement series. Using the sum of differentials routine and re-assigning the zero-displacement reference frame via an external gating signal, such as ECG, is an optional solution to minimize inaccuracy for an analysis time series exceeding the duration of one cardiac cycle, as is described now in Figure 3.

Figure 4 illustrates an exemplary correlation for an amplitude of monitored deformation change of the surface of the heart during a volume therapy according to an advantageous embodiment of the invention. Patients undergoing open heart surgery are routinely artificially ventilated, using a positive (or negative) pressure ventilator device. Adjustment of the ventilator settings with respect to frequency, gas mixture, and especially pressure affects right ventricle function. The most adverse outcome of non-optimal ventilator settings are right ventricular (RV) failure and dysfunction. Ventilator level steering using both amplitude and amplitude modulation values of displacement or strain data delivered by the present invention improves the adjustment of optimum level and reduces the risk of choice of detrimental settings. Ventilator settings can be adjusted either manually based on the indicative parameters, or via an automated feedback control loop, where the targeted performance level of the right ventricle is set, and ventilator settings adjusted incrementally in order to reach the target.

The same principle applies also to an Intravenous Fluid Therapy, which is carried out to prevent dehydration, i.e., deficit of total body water. Fluid replacement is provided by intravenous infusion. Fluid overload occurs when fluids are given at a higher rate or in a larger volume than the system can absorb or excrete. Possible consequences include hypertension, heart failure, and pulmonary edema. The measurements and estimates provided by the embodiments of the present invention can generate accurate information about the effect of the fluid therapy on the patient's heart and indicate conditions of hypo/hyper infusion to the operating team, for instance, by indication of deviation from optimal fractional shortening values or principal strain values. The amplitude representing said deformation is illustrated in Figure 4 in a function of volume, where it can be clearly seen that when the intravenous infusion is increased also the amplitude increased to a certain acceptable level, but beyond a certain point 112, where the fluids are given at a higher rate or in a larger volume than the system can absorb or excrete, the change of the deformation is not in the range or limit anymore and the amplitude representing the change of the deformation changes dramatically. This can be clearly seen in the point 112 in Figure 4, which data can also be used for providing control data or signal to the infusion machine, for example.

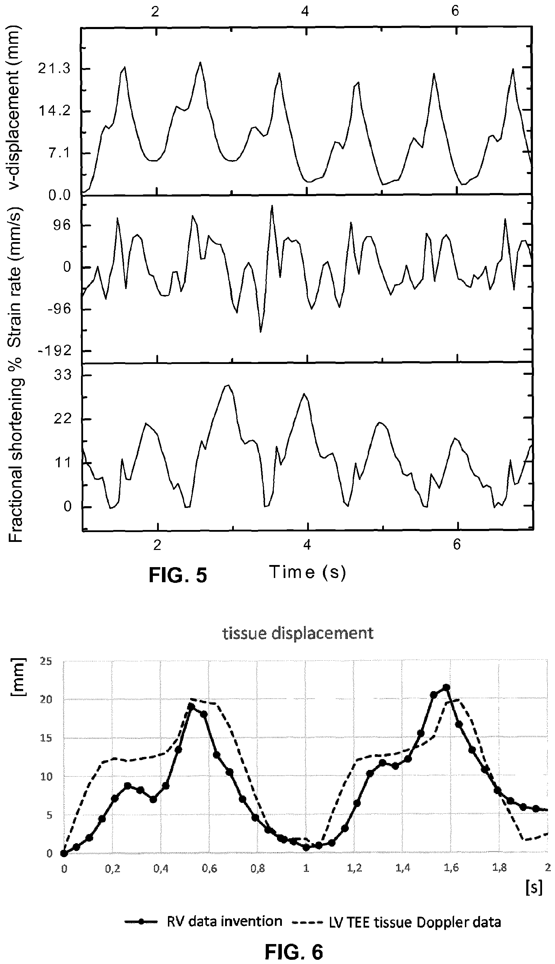

Figures 5-7 illustrate examples of displacement and deformation data time series according to an advantageous embodiment of the invention. By linking the series of images through a common time base to other physiological monitoring measures such as electrocardiography (ECG), heart rate, blood pressure, central venous pressure, pulmonary artery pressure, pulmonary artery occlusion pressure, inspired and expired gases, oxygen saturation of the blood, cardiac output, cerebral activity, neuromuscular function, other ultrasound or tissue doppler imaging data etc., accurate monitoring and quantification of cardiac cycle events in the right ventricle is possible and can be put into context of other vital sings and measures. Extracting displacement data time series functions x→f(t) enables visualization and quantification of deformation pattern characteristics with respect to amplitude, amplitude modulation, periodicity, aperiodicity, wavelength, frequency, phase, rhythm or other features and their combination, which can be interpreted either visually-cognitively by a clinician in respect of clinical characteristics, possibly associated with certain diseases or conditions, or by applying an automated pattern recognition routine, and steer or guide the settings of life support machines, equipment and measures such as cardiopulmonary bypass pump settings, ventilator/ respirator settings (volume, pressure, flow), infusion pumps etc.

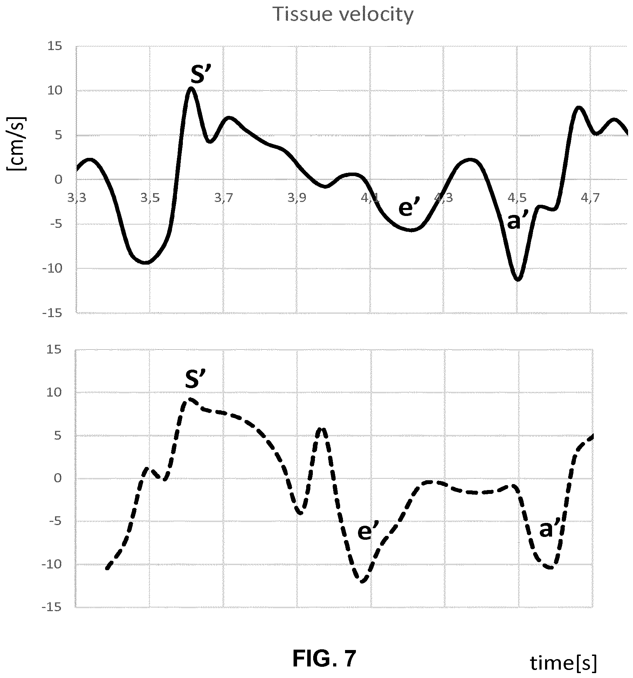

In particular Figures 6 and 7 illustrate tissue displacement and velocity data gathered from the right ventricle (RV) by the current invention and from the left ventricle (LV) by the TEE tissue Doppler method. The waveforms of the invention resemble the waveforms obtained by TEE tissue Doppler, showing the same characteristic cardiac cycle events and amplitudes, whilst acquisition is faster, easier, and of higher accuracy.

The invention has been explained above with reference to the aforementioned embodiments, and several advantages of the invention have been demonstrated. It is clear that the invention is not only restricted to these embodiments, but comprises all possible embodiments within the spirit and scope of the inventive thought and the following patent claims. The features recited in dependent claims are mutually freely combinable unless otherwise explicitly stated.

For example, even if optical or near optical electromagnetic radiation cameras are described as an example, also other type of cameras can be used, such as infrared cameras, whereupon also oxygen saturation can be determined during the same measurements. In addition, the monitoring and assessment can be implemented even with one camera, e.g., of a Plenoptic type. Moreover, even if it is said that at least two images are taken, but in practice typically at least 100 images are taken in order to get better reliability. In addition, the images taken into the process can be consecutive, but also single images from the imaging sequence can be ignored. Still in addition it is to be noted that in accordance to an embodiment also minutes of the operation can be provided with the information and assessment data gathered by the current invention automatically.

In addition, even if the imaging device (102) is described, the arrangement may comprise just an imaging input device for receiving image data from the outer imaging device. Furthermore, even if the at least one surface point 113 is disclosed from which the two or more images are taken, in practise it is a pattern (comprising a number of points), which is imaged. However, it is to be understood that in some situations also one point to be imaged is enough, when e.g. the speed, acceleration and/or direction of said point is determined in function of time (cycles of the heart).

Still, in addition, it is to be noted that according to an embodiment the arrangement can be implemented by the imaging device 102 as such, such as by a camera (advantageously comprising same elements or devices as said arrangement described above, such as described in connection to Fig. 1), wherein said imaging device is configured to obtain at least two or more images of at least one surface point on a surface of the anatomic object over at least one fraction of a cardiac cycle from the surface of the heart. The imaging device comprises advantageously an output for a display device or even comprises said display device. In addition the imaging device comprises a processing unit (or interface to an external processing unit) for determining (advantageously by tracking) deformation based on movements of the at least one surface point over said at least one fraction of the cycle between said at least two or more images of the surface of the anatomic object in function of time. The imaging device may also comprise elements, such as software or hardware elements, for determining changes in the deformation or for comparing determined deformation to reference deformation values to find any deviation in said performance or state of the deformation determined (or a feature changing during a cycle but in a normal state being regular and sequentially repetitive over several cycles), and providing control data for controlling the display device to display the changes in said deformation or said deviation or state of the deformation determined in function of time. As can be understood the imaging device may function as a master imaging device and comprise interface for receiving additional image information from other imaging devices connected with.

Furthermore, even if the examples above are related to the determination of the heart, it is to be understood that also other kinds of anatomic or muscle deformations, such as lungs deformation due to breathing or muscle deformations due to muscle movements, can be tracked and determined in order to monitor and assess the performance of those anatomic objects.

## Claims

1. An arrangement (100) for electromagnetic radiation based non-invasive monitoring or assessment of a performance of an anatomic object (101) during an operation or during medical intervention or during medical examination of a subject,
wherein the arrangement (100) comprises:
- at least one imaging device (102) or imaging input device for obtaining at least two or more images of at least one surface point (113) on a surface of the anatomic object over at least one fraction of a muscle movement cycle from the surface of the anatomic object,
- an output for a display device (106) or said display device (106), and
- a control unit (104) for determining deformation based on movements of said at least one surface point (113) over said at least one fraction of the cycle between said at least two or more images of the surface of the anatomic object in function of time and further determining changes in said deformation or for comparing determined deformation to reference deformation values to find any deviation in said performance or state of the deformation determined, and providing control data for controlling the display device (106) to display the changes in said deformation or said deviation or state of the deformation determined in function of time.

2. The arrangement of claim 1, wherein the anatomic object is a heart, in particular myocardium, or lung, or other skeletal muscle, such as a back muscle or limb muscle.

3. The arrangement of any of previous claims, wherein the deformation is or comprises at least one of the following:
- variable distance of at least two surface points (113) or local displacement of at least one surface point on the surface of the anatomic object, such as a right side of the heart, over said at least one fraction of the cycle, such as a cardiac cycle,
- tissue displacement, velocity, or acceleration of at least one surface point over said at least one fraction of the cycle,
- contractility indicator based on strain rate, shortening fraction, principal shortening strain or other deformation values determined from at least two surface points over said at least one fraction of the cycle, or
- contractility of the heart based on the contractility indicator and simultaneous measurement of pressures in a right atrium or right ventricle with a pulmonary artery catheter or other pressure measurement, and/or
- variable surface area and/or volume defined by at least three surface points on the surface of the anatomic object over said at least one fraction of the cycle.

4. The arrangement of any of previous claims, wherein the control unit (104) is configured to provide displacement vector(s) (110) for at least one surface point (113) on the images as a time series function from the data representing a position of pixel kernels in space for determining said changes in said deformation.

5. The arrangement of any of previous claims, wherein the arrangement is configured to gather time series data from at least one additional external measuring device (103) measuring vital signs of the subject, and wherein the arrangement is configured to provide at least one trigger pulse (111) or a common time base for synchronizing or linking at least two or more images and/or or other measurement data from at least one of said imaging device (102) or other device with each other and therefore determining said changes in said deformation between said two or more images, where said trigger pulse (111) or the common time base is based on said time series data.

6. The arrangement of claim 5, wherein the arrangement is configured to set a zero-displacement reference frame for a first image gated by said trigger pulse (111) or the common time base and compare a displacement vector (110) provided for at least one second subsequent image obtained over at least one next fraction of a cycle to said zero-displacement reference frame and thereby determining said deformation or said changes in said deformation.

7. The arrangement of claim 5 or 6, wherein said trigger pulse (111) or said common time base is based on the other physiological monitoring measures (103) such as electrocardiography (ECG), heart rate, blood pressure, central venous pressure, pulmonary artery pressure, pulmonary artery occlusion pressure, inspired and expired gases, oxygen saturation of the blood, cardiac output, ultrasound or tissue doppler imaging data or other monitoring and quantification of cardiac cycle events in a right ventricle is provided into context of other vital sings and measures.

8. The arrangement of any of previous claims, wherein the arrangement further comprises and/or is arranged to provide control data for controlling life support machines (107), equipment and/or measures, such as cardiopulmonary bypass pump settings, ventilator or respirator settings or infusion pumps so that the deformation state and/or determined changes in said deformation is kept in or adjusted to a predetermined range or value or target range or value is achieved.

9. The arrangement of any of previous claims, wherein the arrangement is configured to visualize and quantify a deformation pattern characteristics with respect to amplitude, amplitude modulation, periodicity, aperiodicity, wavelength, frequency, phase, rhythm and/or combination thereof for representing said deformation or said changes in said deformation.

10. The arrangement of claim 9, wherein the arrangement is configured to determine a time lag between an event of an electrical activity measured by electrocardiography (ECG) and said deformation pattern for representing said deformation or said changes in said deformation.

11. The arrangement of any of previous claims, wherein the control unit (104) is configured to provide a trend line control data for controlling the display device (106) to display a trend line, said trend line representing a trend of changes in said deformation.

12. The arrangement of any of previous claims, wherein the control unit (104) is configured to compare the value of the deformation and/or a trend of the changes in said deformation to a predetermined value(s) or range, and whether said determined deformation or a trend deviates from the predetermined value or range, the control unit (104) is configured to provide an alarm control data for controlling the display device (106) or other device to display or generate the alarm indicating the deviating values and/or changes in said deformation.

13. The arrangement of any of previous claims, wherein the arrangement comprises or is at least arranged in a data communication (108) with a database (109) and is configured to provide data related to said deformation and/or changes in said deformation to said database and/or wherein said arrangement is configured to compare said determined deformation and/or changes in said deformation data to data previously stored in said database and thereby configured to identify possible match with said previously stored data and found a possible association with certain diseases or conditions inducing said deformation and/or deviating changes in said deformation.

14. The arrangement of any of previous claims, wherein the arrangement is configured to determine the state of said deformation and/or changes in said deformation by identifying common points or pixel kernels on images captured by at least two different imaging devices (102) at the same point in time, and by tracing on each image of an imaging time sequence, where said tracing or a correlation is performed on images.

15. The arrangement of any of previous claims, wherein the arrangement is additionally configured to determine two- or three-dimensional coordinates of points on the surface of the anatomic object by measurement data obtained from the imaging device(s) using a coordinate system transformation.

16. The arrangement of any of previous claims, wherein the arrangement comprises a pattern providing device for providing a pattern on the surface of the anatomic object.

17. The arrangement of any of previous claims, wherein the arrangement further comprises and/or is arranged to receive a trigger signal from an ECG monitor and start data acquisition in case of detecting abnormal electrical activity.

18. An imaging device (102) for electromagnetic radiation based non-invasive monitoring or assessment of a performance of an anatomic object (101) during an operation or during medical intervention or during medical examination of a subject, wherein
- said imaging device (102) configured to obtain at least two or more images of at least one surface point (113) on a surface of the anatomic object over at least one fraction of a cycle from the surface of the anatomic object,
wherein the imaging device comprises:
- an output for a display device (106) or said display device, and
- a control unit (104) for determining deformation based on movements of said at least one surface point (113) over said at least one fraction of the cycle between said at least two or more images of the surface of the anatomic object in function of time and further determining changes in said deformation or for comparing determined deformation to reference deformation values to find any deviation in said performance or state of the deformation determined, and providing control data for controlling the display device (106) to display the changes in said deformation or said deviation or state of the deformation determined in function of time.

19. A method for electromagnetic radiation based non-invasive monitoring or assessment of a performance of an anatomic object (101) during an operation or during medical intervention or during medical examination of a subject,
wherein the method comprises steps of:
- obtaining at least two or more images of at least one surface point (113) on a surface of the anatomic object (101) over at least one fraction of a cycle from the surface of the anatomic object, and
- determining deformation based on movements of said at least one surface point (113) over said at least one fraction of the cycle between said at least two or more images of the surface of the anatomic object in function of time and further determining changes in said deformation or for comparing determined deformation to reference deformation values to find any deviation in said performance or state of the deformation determined.

20. A computer program product adapted to monitor or asses a performance of an anatomic object (101) during an operation or during medical intervention or during medical examination of a subject,
wherein the computer program product is adapted to perform steps of:
- obtaining at least two or more images of at least one surface point (113) on a surface of the anatomic object over at least one fraction of a cycle from the surface of the heart, and
- determining deformation based on movements of said at least one surface point (113) over said at least one fraction of the cycle between said at least two or more images of the surface of the anatomic object in function of time and further determining changes in said deformation or for comparing determined deformation to reference deformation values to find any deviation in said performance or state of the deformation determined,
when said computer program product is run on a data processing device.
